# EUROPÄISCHE PATENTSCHRIFT

(11) **EP 2 800 733 B1**
(45) Veröffentlichungstag und Bekanntmachung des Hinweises auf die Patenterteilung: **06.04.2016**
(21) Anmeldenummer: 13700056.8
(22) Anmeldetag: 02.01.2013
(51) Int. Cl.: C07C 7/08, C07C 11/167

(54) **VERFAHREN ZUR AUFREINIGUNG EINES RECYCLE-STROMES AUS EINER 1,3-BUTADIEN VERARBEITENDEN ANLAGE**
PROCESS FOR PURIFYING A RECYCLE STREAM FROM AN INSTALLATION PROCESSING 1,3-BUTADIENE
PROCÉDÉ DE PURIFICATION D'UN COURANT DE RECYCLAGE D'UNE INSTALLATION DE TRANSFORMATION DE 1,3-BUTADIÈNE

(30) Priorität: 03.01.2012 EP 12150073
(43) Veröffentlichungstag der Anmeldung: 12.11.2014
(73) Patentinhaber: BASF SE, 67056 Ludwigshafen am Rhein (DE)
(72) Erfinder: HEIDA, Bernd, 67158 Ellerstadt (DE); HUGO, Randolf, 67246 Dirmstein (DE)
(74) Vertreter: BASF IP Association
(86) Internationale Anmeldenummer: PCT/EP2013/050002
(87) Internationale Veröffentlichungsnummer: WO 2013/102625

(56) Entgegenhaltungen:
- WO-A1-2010/134955
- US-A- 3 798 132

## Beschreibung

Die Erfindung betrifft ein Verfahren zur Aufreinigung eines Recycle-Stromes aus einer 1,3-Butadien verarbeitenden Anlage.

In 1,3-Butadien verarbeitenden Anlagen fallen häufig Nebenströme an, die noch hohe Gehalte an dem Wertprodukt 1,3-Butadien aufweisen. Diese Ströme wurden bislang extern aufgearbeitet oder entsorgt.

Die Dokumente US 3,798,132 und WO 2010/134955 offenbaren die Aufereinigung eines 1,3-Butadien enthaltenden Strom durch Extraktivdestillation.

Es war daher Aufgabe der Erfindung, ein Verfahren zur Verfügung zu stellen, das es ermöglicht, Nebenströme aus 1,3-Butadien verarbeitenden Anlagen, die noch hohe Gehalte an Wertprodukt 1,3-Butadien aufweisen, in einer verfahrenstechnisch einfachen und energetisch vorteilhaften Weise so weit aufzureinigen, dass polymerisationsfähiges 1,3-Butadien erhalten wird, das den Spezifikationsanforderungen für den Feed-Strom in die 1,3-Butadien verarbeitende Anlage genügt, so dass dieser als Recycle-Strom in der Anlage selbst genutzt werden kann. Das Verfahren ist darüber hinaus auch unter ökologischen Gesichtspunkten vorteilhaft.

Die Lösung besteht in einem Verfahren zur Aufreinigung eines Recycle-Stromes aus einer 1,3-Butadien verarbeitenden Anlage, enthaltend
- 70 - 99 Gew.-% 1,3-Butadien, bezogen auf das Gesamtgewicht des Recycle-Stroms,
- 0,1 - 10 Gew.% Leichtsieder gegenüber 1,3-Butadien, bezogen auf das Gesamtgewicht des Recycle-Stroms und
- 0,1-20 Gew.-% Hochsieder gegenüber 1,3-Butadien, bezogen auf das Gesamtgewicht des Recycle-Stroms, wobei
die Summe an 1,3-Butadien, Leichtsiedern und Hochsiedern im Recycle-Strom 100 Gew.% beträgt und
von einem Rein-1,3-Butadien-Feedstrom, mit einem 1,3-Butadiengehalt von größer oder gleich 98 Gew.-% 1,3-Butadien, bezogen auf das Gesamtgewicht des Rein-1,3-Butadien-Feedstromes, ausgegangen wird, der vorab einer Vorreinigung zur Abtrennung von Hochsiedern gegenüber 1,3-Butadien in einer Abtriebskolonne unterworfen und anschließend der 1,3-Butadien verarbeitenden Anlage zugeführt wird, das dadurch gekennzeichnet ist, dass
- der Recycle-Strom in zwei Teilströme aufgeteilt wird, und zwar in
- einen ersten Teilstrom, der in der Abtriebskolonne, zur Vorreinigung des Rein-1,3-Butadien-Feedstromes aufgereinigt wird, unter Erhalt eines ersten gereinigten Recycle-Stromes, der den vorgereinigten Rein-1,3-Butadien-Feedstrom mit umfasst, sowie
- einen zweiten Teilstrom, der in einer Extraktivdestillationsanlage durch Extraktivdestillation mit einem selektiven Lösungsmittel aufgereinigt wird, wobei die Extraktivdestillationsanlage
   - einen Hauptwäscher umfasst, der als Verstärkungskolonne ausgebildet ist und dem der zweite Teilstrom des Recycle-Stromes im unteren Bereich und das selektive Lösungsmittel im oberen Bereich desselben flüssig zugeführt wird, wobei das selektive Lösungsmittel im Gegenstrom zum zweiten Teilstrom des Recycle-Stromes durch den Hauptwäscher strömt und sich dabei mit 1,3-Butadien belädt, und aus dem ein Kopfstrom, enthaltend Butane und Butene, ausgeschleust wird, sowie
   - eine Ausgaserkolonne, der der Sumpfstrom aus dem Hauptwäscher zugeführt wird, und aus der über Kopf ein zweiter gereinigter Recycle-Strom, sowie ein Sumpfstrom, enthaltend gereinigtes selektives Lösungsmittel abgezogen werden, und wobei
der erste gereinigte Recycle-Strom und der zweite gereinigte Recycle-Strom in die 1,3-Butadien verarbeitende Anlage zurückgeführt werden,
mit der Maßgabe, dass die Aufteilung des Recycle-Stromes in der Weise erfolgt, dass ein möglichst großer Mengenstrom auf den ersten Teilstrom entfällt, unter Einhaltung der Spezifikationsvorgaben für die Summe aller in die 1,3-Butadien verarbeitende Anlage zugeführten Ströme, von einem Anteil an 1,3-Butadien von größer oder gleich 90 Gew.-%, bezogen auf das Gesamtgewicht aller C4-Kohlenwasserstoffe der Summe aller in die 1,3-Butadien verarbeitende Anlage zugeführten Ströme.

Es wurde gefunden, dass es möglich ist, einen Recycle-Strom aus einer 1,3-Butadien verarbeitenden Anlage, der noch einen relativ hohen Anteil, im Bereich von 70 bis 99 Gew.-%, 1,3-Butadien enthält, in verfahrenstechnisch günstiger Weise aufzuarbeiten, so dass ein möglichst großer Teilstrom desselben in einfacher Weise, in einer Abtriebskolonne, von Hochsiedern abgereinigt wird und wobei die wesentlich aufwändigere Reinigung, durch Extraktivdestillation unter Einsatz eines selektiven Lösungsmittels, lediglich für den restlichen Teilstrom erforderlich ist, der möglichst klein gewählt wird. Dabei wird die Aufteilung auf die beiden Teilströme dergestalt vorgenommen, dass die Spezifikationsvorgaben für den Feed-Strom in die 1,3-Butadien verarbeitende Anlage erfüllt werden.

Der Recycle-Strom, der als Nebenstrom aus einer 1,3-Butadien verarbeitenden Anlage anfällt, enthält typischerweise zwischen 70 und 99 Gew.-% 1,3-Butadien, bezogen auf das Gesamtgewicht des Recycle-Stroms, 0,1 bis 20 Gew.-% Leichtsieder gegenüber 1,3-Butadien, bezogen auf das Gesamtgewicht des Recycle-Stroms, 0,1 bis 20.Gew.% Hochsieder gegenüber 1,3-Butadien, bezogen auf das Gesamtgewicht des Recycle-Stroms, wobei die Summe an 1,3-Butadien, Leichtsiedern und Hochsiedern im Recycle-Strom 100 Gew.% beträgt.

Bevorzugt liegt der Gehalt an 1,3-Butadien im Recycle-Strom im Bereich von 80 bis 95 Gew.-%, bezogen auf das Gesamtgewicht des Recycle-Stromes.

Leichtsieder gegenüber 1,3-Butadien sind vorliegend insbesondere C₁ bis C₃-Kohlenwasserstoffe.

Als Leichtsieder gegenüber 1,3-Butadien enthält der Recycle-Strom insbesondere Methan, bevorzugt in einem Anteil von größer oder gleich 90 Gew.-%, bezogen auf das Gesamtgewicht der Leichtsieder gegenüber 1,3-Butadien.

Unter einem Rein-1,3-Butadien-Feedstrom wird vorliegend ein Strom verstanden, der einen 1,3-Butadiengehalt von größer oder gleich 98 Gew.-%, bezogen auf das Gesamtgewicht des Rein-1,3-Butadien-Feedstroms, aufweist.

Weiter bevorzugt wird als Rein-1,3-Butadien handelsübliches Rein-Butadien mit mindestens 99,0 Gew.-% 1,3-Butadien oder auch mindestens 99,4 Gew.-% 1,3-Butadien, oder auch mindestens 99,75 Gew.-% 1,3-butadien, jeweils bezogen auf das Gesamtgewicht, verstanden.

Dieser wird vor der Zuführung zur 1,3-Butadien verarbeitenden Anlage einer Vorreinigung zur Abtrennung von Hochsiedern gegenüber 1,3-Butadien in einer Abtriebskolonne unterworfen.

Die Abtriebskolonne wird bevorzugt bei erhöhtem Druck, insbesondere im Bereich von 3,5 bis 6,0 bar absolut betrieben. Die Abtriebskolonne ist bevorzugt mit trennwirksamen Einbauten bestückt, insbesondere mit Böden.

Erfindungsgemäß wird dieser, bereits für die Vorreinigung des Rein-1,3-Butadien-Feedstromes vorhandenen Abtriebskolonne zusätzlich zum Rein-1,3-Butadien-Feedstrom auch ein möglichst großer Teilstrom des Recycle-Stromes aus der 1,3-Butadien verarbeitenden Anlage zugeführt. Auch dieser Strom wird, wie auch der Rein-1,3-Butadien-Feedstrom, im oberen Bereich der Abtriebskolonne zugeführt. Die Kolonne muss hierzu lediglich an den höheren Durchsatz angepasst werden.

Der verbleibende, zweite Teil des Recycle-Stromes wird einer vereinfachten Extraktivdestillationsanlage zugeführt, worin die Aufreinigung desselben unter Zusatz eines selektiven Lösungsmittels durchgeführt wird. Die Extraktivdestillationsanlage ist insofern vereinfacht, als es im Normalfall ausreicht, den zweiten Teilstrom des Recycle-Stromes einem Hauptwäscher zuzuführen, der als Verstärkungskolonne ausgebildet ist, wobei auf einen Abtriebsteil verzichtet wird.

Dem Hauptwäscher werden der zweite Teilstrom des Recycle-Stromes im unteren Bereich und das selektive Lösungsmittel im oberen Bereich desselben zugeführt. Aus dem Hauptwäscher wird ein Kopfstrom, enthaltend Butane und Butene abgeschleust und ein Sumpfstrom abgezogen, der ein mit 1,3-Butadien beladenes selektives Lösungsmittel enthält.

Der Hauptwäscher ist als Destillationskolonne ausgeführt, mit einer Anzahl von theoretischen Trennstufen, die stark mit dem eingesetzten Lösungsmittel und den geforderten Reinheiten des 1,3-Butadiens variieren kann. Typischerweise können dies 5 bis 50, weiter bevorzugt 12 bis 30 theoretische Trennstufen sein.

Der Kopfdruck im Hauptwäscher liegt bevorzugt im Bereich von 3,5 bis 6,0 bar absolut. Die Temperatur am Kolonnenkopf des Hauptwäschers liegt bevorzugt im Bereich von 20 bis 40 °C.

Die Ausgaserkolonne wird bei gegenüber dem Hauptwäscher niedrigerem Druck betrieben.

Als selektives Lösungsmittel in der Extraktivdestillation werden bevorzugt die üblichen für die Abtrennung von 1,3-Butadien bekannten Lösungsmittel eingesetzt.

Geeignete selektive Lösungsmittel für das erfindungsgemäße Verfahren sind zum Beispiel Butyrolacton, Nitrile wie Acetonitril Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmörpholin, N-alkylsubstituierte cyclische Säureamide (Lactame) wie N-Alkylpyrrolidone, insbesondere N-Methylpyrrolidon. Im Allgemeinen werden N-alkylsubstituierte niedere aliphatische Säureamide oder N-alkylsubstituierte cyclische Säureamide verwendet. Besonders vorteilhaft sind Dimethylformamid, Acetonitril, Furfurol und insbesondere N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Colösungsmitteln wie Wasser und/oder tert.-Butylether, zum Beispiel Methyl-tert.-butylether, Ethyl-tert.-butylether, Propyl-tert.-butylether, n- oder iso-Butyl-tert.-butylether eingesetzt werden.

Besonders geeignet ist N-Methylpyrrolidon, bevorzugt in wässriger Lösung, insbesondere mit 8 bis 10 Gew.-% Wasser, besonders bevorzugt mit 8,3 Gew.-% Wasser.

Erfindungsgemäß wird der Recycle-Strom aus der 1,3-Butadien verarbeitenden Anlage in der Weise auf die beiden Teilanlagen zur Aufarbeitung desselben aufgeteilt, dass der Teilstrom, der zusammen mit der Vorreinigung des Rein-1,3-Butadien-Feed-Stromes lediglich destillativ, in einer Abtriebskolonne, aufgereinigt wird, möglichst groß ist und dass der zweite, restliche Teilstrom, lediglich so groß gewählt wird, dass die Spezifikationsanforderungen für die Summe aller in die 1,3-Butadien verarbeitende Anlage zugeführten Ströme (Feed-Ströme) eingehalten werden.

Die Aufarbeitung des zweiten Teilstromes ist technisch und energetisch aufwändiger, als die Aufarbeitung des ersten Teilstroms, da es sich hierbei um eine Extraktivdestillation handelt. Auch diese Extraktivdestillationsanlage ist jedoch erfindungsgemäß gegenüber dem Stand der Technik vereinfacht: Der Hauptwäscher, dem der Teilstrom, des Recycle-Stromes im Gegenstrom zum selektiven Lösungsmittel zugeführt wird, wobei sich das selektive Lösungsmittel mit 1,3-Butadien belädt, ist erfindungsgemäß als Verstärkungskolonne ausgebildet, das heißt der dem Hauptwäscher als Feed-Strom zugeführte zweite Teilstrom des Recycle-Stromes wird dem Hauptwäscher im unteren Bereich desselben zugeführt. Der Hauptwäscher weist somit kein Abtriebsteil auf.

Falls erforderlich, kann die Extraktivdestillationsanlage in einer besonderen Ausführungsvariante eine dem Hauptwäscher vorgeschaltete Abtriebskolonne zur Abtrennung von Schwersiedern aus dem zweiten Teilstrom des Recycle-Stromes vor der Zuführung desselben zum Hauptwäscher aufweisen.

Die Ausgaserkolonne der Extraktivdestillationsanlage wird bevorzugt dergestalt ausgelegt, dass der zweite gereinigte Recycle-Strom, der über Kopf desselben abgezogen wird, einen 1,3-Butadien Gehalt von > 96 Gew.-%, bevorzugt von > 99,5 Gew.-%, bezogen auf das Gesamtgewicht des zweiten gereinigten Recycle-Stromes, aufweist.

Die Abtriebskolonne wird bevorzugt dergestalt ausgelegt, dass aus dem ersten gereinigten Recycle-Strom, der über Kopf derselben abgezogen wird, die Hochsieder bis auf einen Restgehalt von maximal 1000 ppm abgetrennt werden.

In einer weiteren bevorzugten Verfahrensvariante wird aus dem Recycle-Strom aus der 1,3-Butadien verarbeitenden Anlage ein weiterer Teilstrom abgetrennt, der ohne Vorreinigung in die 1,3-Butadien verarbeitende Anlage recycliert wird.

Dieser Teilstrom ist bevorzugt größer als die Hälfte des gesamten Recycle-Stromes.

Die 1,3-Butadien verarbeitende Anlage ist insbesondere eine Anlage zur Polymerisation von 1,3-Butadien oder zur Copolymerisation von 1,3-Butadien mit einem oder mehreren Comonomeren.

Comonomere sind insbesondere Styrol und/oder Acrylnitril.

Die Polymerisation oder Copolymerisation des 1,3-Butadiens kann insbesondere eine Lösungspolymerisation sein.

Die Lösungspolymerisation kann insbesondere in Gegenwart von Toluol oder Hexan als Lösungsmittel durchgeführt werden.

Die destillative Abtrennung von 1,3-Butadien aus einem C₄-Kohlenwassergtoffe enthaltenden Gemisch ist verfahrenstechnisch schwierig, weil sich die Siedepunkte der C₄-Kohlenwasserstoffe nur wenig voneinander unterscheiden. Erst durch Zugabe eines selektiven Lösungsmittels wird die Auftrennung in der Regel mit vertretbarem Aufwand möglich.

Es ist daher umso überraschender, dass durch die erfindungsgemäße Verfahrensführung eine Aufreinigung und Rückführung von in 1,3-Butadien verarbeitenden Anlagen anfallenden Strömen, die die Voraussetzung erfüllen, dass die Summe der in die 1,3-Butadien verarbeitende Anlage zugeführten Ströme einen 1,3-Butadien Anteil von ≥ 90 Gew.-% aufweisen, erreicht werden kann, indem ein möglichst großer Teilstrom einer verfahrenstechnisch einfachen und energetisch wenig aufwändigen klassischen Destillation in einer Abtriebskolonne zugeführt wird.

Die nachfolgenden Berechnungen verdeutlichen das erhebliche Energieeinsparpotential der Verfahrensführung nach der Erfindung gegenüber der herkömmlichen Verfahrensweise:

Hierfür wurde der Verbrauch in kJ/kg pro aufgearbeitetes 1,3-Butadien nach unterschiedlichen Verfahrensvarianten berechnet:

### Spezifischer Energieverbrauch nach dem Stand der Technik:

Nach dem Stand der Technik (herkömmliche Extraktivdestillation), wobei eine weitgehende Wärmeintegration bereits berücksichtigt ist, beträgt der spezifische Energieverbrauch 3898 kJ/kg 1,3-Butadien. Hinzu kommt die Verdampfungswärme für 1,3-Butadien von ca. 370 kJ/kg, in Summe also 4268 kJ/kg.

### Spezifischer Energieverbrauch nach dem erfindungsgemäßen Verfahren:

Für die Aufarbeitung des ersten Teilstromes (erfindungsgemäß) in der Abtriebskolonne K1 (ohne Sumpfverdampfer) wird lediglich die Verdampfungswärme für 1,3-Butadien, von ca. 370 kJ/kg, benötigt.

Zur Aufarbeitung des zweiten Teilstroms (erfindungsgemäß) in einer vereinfachten Extraktivdestillationsanlage, mit einem Hauptwäscher als Verstärkungskolonne, sind für die Vorverdampfung 386 kJ/kg erforderlich plus 2039 kJ/kg für die Extraktivdestillation im Hauptwäscher und in der Ausgaserkolonne, also in Summe 2425 kJ/kg. Ein Teil der Wärme (etwa 70 %) lässt sich durch Wärmeintegration zurückgewinnen. Übrig bleiben für die Extraktivdestillation 619 kJ/kg. Hinzu kommen für die Vörverdampfung 386 kJ/kg. In Summe werden für diese Verfahrensvariante ca. 1000 kJ/kg benötigt, das heißt weniger als ein Viertel gegenüber einer herkömmlichen Extraktivdestillation.

Indem erfindungsgemäß ein möglichst großer Anteil des Recycle-Stroms auf den ersten Teilstrom aufgeteilt wird, ist der Energieverbrauch im erfindungsgemäßen Verfahren entsprechend noch deutlich niedriger als ein Viertel des Verbrauchs in einer herkömmlichen Extraktivdestillation.

Die Erfindung wird im Folgenden anhand einer Zeichnung verdeutlicht.

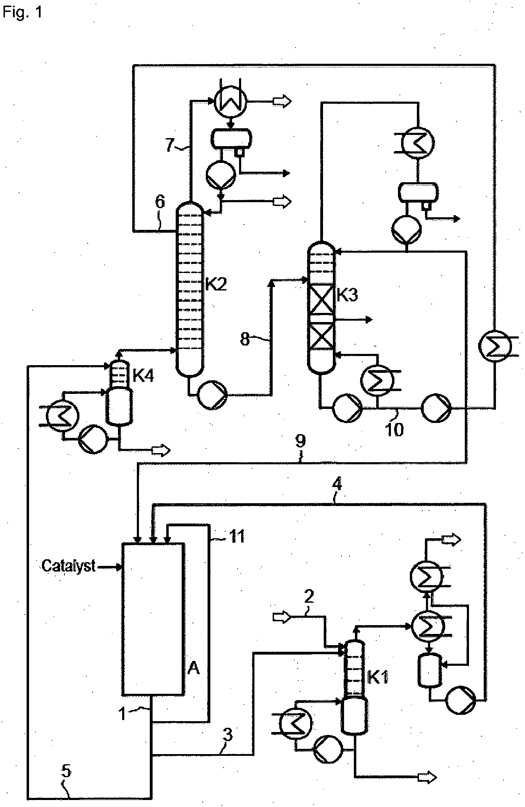

Fig. 1 zeigt die schematische Darstellung einer bevorzugten Anlage zur Durchführung des erfindungsgemäßen Verfahrens.

Einer 1,3-Butadien verarbeitende Anlage (A) wird ein 1,3-Butadien-Feedstrom (2) zugeführt, der vorab einer Vorreinigung in einer Abtriebskolonne (K1) zugeführt wurde, aus der über Sumpf Hochsieder ausgeschleust werden.

Aus der 1,3-Butadien verarbeitenden Anlage (A) wird ein Recycle-Strom (1) abgezogen, der in einen ersten Teilstrom (3) aufgeteilt wird, der ebenfalls der Abtriebskolonne (K1) zugeführt wird, aus der Hochsieder ausgeschleust werden und aus der ein Kopfstrom abgezogen wird, der als erster gereinigter Recycle-Strom (4) der 1,3-Butadien verarbeitende Anlage (A) zugeführt wird.

Aus dem Recycle-Strom (1) wird ein zweiter Teilstrom (5) abgetrennt, der einer Extraktivdestillationsanlage zugeführt wird. In der in Figur 1 dargestellten bevorzugten Ausführungsvariante wird der zweite Teilstrom (5) des Recycle-Stromes (1) vorab einer Abtriebskolonne (K4) zur Abtrennung von Hochsiedern und anschließend dem Hauptwäscher (K2), der als Verstärkungskolonne ausgebildet ist, im unteren Bereich desselben, zugeführt. Dem Hauptwäscher (K2) wird im oberen Bereich desselben selektives Lösungsmittel (Strom 6) zugeführt.

Über Kopf des Hauptwäschers (K2) wird ein Strom (7) abgezogen, der Butane und Butene enthält, und der ausgeschleust wird.

Der Sumpfstrom aus dem Hauptwäscher (K2) wird der Ausgaserkolonne (K3) zugeführt, aus der ein Sumpfstrom (10), enthaltend gereinigtes selektives Lösungsmittel abgezogen wird, das in den Hauptwäscher (K2) recycliert wird und ein Kopfstrom, aus dem ein zweiter gereinigter Recycle-Strom (9) in die 1,3-Butadien verarbeitende Anlage recycliert wird.

In der in Figur 1 dargestellten bevorzugten Verfahrensvariante ist ein dritter Teilstrom des Recycle-Stromes (1) vorgesehen, Strom 11, der unmittelbar, ohne weitere Aufreinigung, in die 1,3-Butadien verarbeitende Anlage (A) recycliert wird.

## Patentansprüche

1. Verfahren zur Aufreinigung eines Recycle-Stromes (1) aus einer 1,3-Butadien verarbeitenden Anlage (A), enthaltend
- 70 - 99 Gew.-% 1,3-Butadien, bezogen auf das Gesamtgewicht des Recycle-Stroms (1),
- 0,1 - 10 Gew.% Leichtsieder gegenüber 1,3-Butadien, bezogen auf das Gesamtgewicht des Recycle-Stroms (1) und
- 0,1 - 20 Gew.-% Hochsieder gegenüber 1,3-Butadien, bezogen auf das Gesamtgewicht des Recycle-Stroms (1), wobei
die Summe an 1,3-Butadien, Leichtsiedern und Hochsiedern im Recycle-Strom 100 Gew.-% beträgt und
von einem Rein-1,3-Butadien-Feedstrom (2), mit einem 1,3-Butadiengehalt von größer als 98 Gew.-% 1,3-Butadien, bezogen auf das Gesamtgewicht des Rein-1,3-Butadien-Feedstromes (2) ausgegangen wird, der vorab einer Vorreinigung zur Abtrennung von Hochsiedern gegenüber 1,3-Butadien in einer Abtriebskolonne (K1) unterworfen und anschließend der 1,3-Butadien verarbeitenden Anlage (A) zugeführt wird, **dadurch gekennzeichnet, dass**
der Recycle-Strom (1) in zwei Teilströme aufgeteilt wird, und zwar in
- einen ersten Teilstrom (3), der in der Abtriebskolonne (K1) zur Vorreinigung des Rein-1-,3-Butadien-Feedstromes (2) aufgereinigt wird, unter Erhalt eines ersten gereinigten Recycle-Stromes (4) der den vorgereinigten Rein-1,3-Butadien-Feedstrom (2) mit umfasst, sowie
- einen zweiten Teilstrom (5), der in einer Extraktivdestillationsanlage durch Extraktivdestillation mit einem selektiven Lösungsmittel (6) aufgereinigt wird, wobei die Extraktivdestillationsanlage
- einen Hauptwäscher (K2) umfasst, der als Verstärkungskolonne ausgebildet ist und dem der zweite Teilstrom (5) des Recycle-Stromes im unteren Bereich und das selektive Lösungsmittel (6) im oberen Bereich desselben flüssig zugeführt wird, wobei das selektive Lösungsmittel (6) im Gegenstrom zum zweiten Teilstrom (5) des Recycle-Stromes durch den Hauptwäscher (K2) strömt und sich dabei mit 1,3-Butadien belädt, und aus dem ein Kopfstrom (7), enthaltend Butane und Butene, ausgeschleust wird, sowie
- eine Ausgaserkolonne (K3), der der Sumpfstrom (8) aus dem Hauptwäscher (K2) zugeführt wird, und aus der über Kopf ein zweiter gereinigter Recycle-Strom (9), sowie ein Sumpfstrom (10), enthaltend gereinigtes selektives Lösungsmittel abgezogen werden, und wobei
der erste gereinigte Recycle-Strom (4) und der zweite gereinigte Recycle-Strom (9) in die 1,3-Butadien verarbeitende Anlage (A) zurückgeführt werden,
mit der Maßgabe, dass die Aufteilung des Recycle-Stromes (1) in der Weise erfolgt, dass ein möglichst großer Mengenstrom auf den ersten Teilstrom (3) entfällt, unter Einhaltung der Spezifikationsvorgaben für die Summe aller in die 1,3-Butadien verarbeitende Anlage (A) zugeführten Ströme, von einem Anteil von 1,3-Butadien von größer oder gleich 90 Gew.-%, bezogen auf das Gesamtgewicht aller C₄-Kohlenwasserstoffe aus der Summe aller in die 1,3-Butadien verarbeitende Anlage (A) zugeführten Ströme.

2. Verfahren nach Anspruch 1, **dadurch gekennzeichnet, dass** die Aufteilung des Recycle-Stromes (1) in der Weise erfolgt, dass der erste Teilstrom (3) größer als der zweite Teilstrom (5) ist.

3. Verfahren nach Anspruch 1 oder 2, **dadurch gekennzeichnet, dass** die 1,3-Butadien verarbeitende Anlage (A) eine Anlage zur Polymerisation von 1,3-Butadien oder zur Copolymerisation, von 1,3-Butadien mit einem oder mehreren Comonomeren, ist.

4. Verfahren nach Anspruch 3, **dadurch gekennzeichnet, dass** als Comonomer(e) Styrol und/oder Acrylnitril eingesetzt werden.

5. Verfahren nach einem der Ansprüche 1 bis 4, **dadurch gekennzeichnet, dass** der Recyclestrom (1) als Leichtsieder gegenüber 1,3-Butadien Methan, insbesondere in einem Anteil von ≥ 90 Gew.-%, bezogen auf das Gesamtgewicht der weiteren Leichtsieder gegenüber 1,3-Butadien, enthält.

6. Verfahren nach einem der Ansprüche 3 bis 5, **dadurch gekennzeichnet, dass** die Polymerisation oder Copolymerisation des 1,3-Butadiens eine Lösungspolymerisation, insbesondere in Gegenwart von Toluol oder Hexan als Lösungsmittel, ist.

7. Verfahren nach einem der Ansprüche 1 bis 6, **dadurch gekennzeichnet, dass** die Abtriebskolonne (K1) dergestalt ausgelegt ist, dass aus dem ersten gereinigten Recycle-Strom (4), der über Kopf derselben abgezogen wird, die Hochsieder bis auf einen Restgehalt von maximal 1000 ppm abgetrennt werden.

8. Verfahren nach einem der Ansprüche 1 bis 7, **dadurch gekennzeichnet, dass** die Ausgaserkolonne (K3) dergestalt ausgelegt ist, dass der zweite gereinigte Recycle-Strom (9), der über Kopf derselben abgezogen wird, einen 1,3-Butadien-Gehalt von größer oder gleich 96 Gew.-%, bevorzugt von größer oder gleich 99,5 Gew.-%, bezogen auf das Gesamtgewicht des zweiten gereinigten Recycle-Stroms (9), aufweist.

9. Verfahren nach einem der Ansprüche 1 bis 8, **dadurch gekennzeichnet, dass** die Extraktivdestillationsanlage eine dem Hauptwäscher (K2) vorgeschaltete Abtriebskolonne (K4) zur Abtrennung von Hochsiedern aus dem zweiten Teilstrom (5) des Recycle-Stromes (1) aufweist.

10. Verfahren nach einem der Ansprüche 1 bis 9, **dadurch gekennzeichnet, dass** aus dem Recycle-Strom (1) ein weiterer Teilstrom (11) abgetrennt wird, der ohne Vorreinigung in die 1,3-Butadien verarbeitende Anlage (A) recycliert wird.

11. Verfahren nach Anspruch 10, **dadurch gekennzeichnet, dass** der weitere Teilstrom (11) größer als die Hälfte des gesamten Recycle-Stromes (1) ist.

## Claims

1. A process for purifying a recycle stream (1) from a 1,3-butadiene processing plant (A), comprising
- 70-99% by weight of 1,3-butadiene, based on the total weight of the recycle stream (1),
- 0.1-10% by weight of low boilers relative to 1,3-butadiene, based on the total weight of the recycle stream (1), and
- 0.1-20% by weight of high boilers relative to 1,3-butadiene, based on the total weight of the recycle stream (1), where
the sum of 1,3-butadiene, low boilers and high boilers in the recycle stream is 100% by weight and
the starting material is a pure 1,3-butadiene feedstream (2) having a 1,3-butadiene content of greater than 98% by weight of 1,3-butadiene, based on the total weight of the pure 1,3-butadiene feedstream (2), which is subjected beforehand to a prepurification for removal of high boilers relative to 1,3-butadiene in a stripping column (K1) and is then supplied to the 1,3-butadiene processing plant (A), which comprises
dividing the recycle stream (1) into two substreams, specifically into
- a first substream (3) which is purified in the stripping column (K1) for prepurification of the pure 1,3-butadiene feedstream (2) to obtain a first purified recycle stream (4) also comprising the prepurified pure 1,3-butadiene feedstream (2), and
- a second substream (5) which is purified in an extractive distillation plant by extractive distillation with a selective solvent (6), said extractive distillation plant comprising
- a main scrubber (K2) which is in the form of a rectifying column and to which are fed in liquid form the second substream (5) of the recycle stream in the lower region and the selective solvent (6) in the upper region, the selective solvent (6) flowing through the main scrubber (K2) in countercurrent to the second substream (5) of the recycle stream and becoming laden with 1,3-butadiene, and from which a top stream (7) comprising butanes and butenes is discharged, and
- an outgasser column (K3) to which the bottom stream (8) from the main scrubber (K2) is supplied, and from which a second purified recycle stream (9) overhead, and a bottom stream (10) comprising purified selective solvent are drawn off, and
the first purified recycle stream (4) and the second purified recycle stream (9) being recycled into the 1,3-butadiene processing plant (A),
with the proviso that the recycle stream (1) is divided in such a way that a maximum flow rate is accounted for by the first substream (3) while complying with the specifications for the sum of all streams fed into the 1,3-butadiene processing plant (A), of a proportion of 1,3-butadiene of greater than or equal to 90% by weight, based on the total weight of all C₄ hydrocarbons from the sum of all streams fed into the 1,3-butadiene processing plant (A).

2. The process according to claim 1, wherein the recycle stream (1) is divided in such a way that the first substream (3) is larger than the second substream (5).

3. The process according to claim 1 or 2, wherein the 1,3-butadiene processing plant (A) is a plant for polymerization of 1,3-butadiene or for copolymerization of 1,3-butadiene with one or more comonomers.

4. The process according to claim 3, wherein the comonomer(s) used is/are styrene and/or acrylonitrile.

5. The process according to any of claims 1 to 4, wherein the recycle stream (1) comprises, as low boilers relative to 1,3-butadiene, methane, especially in a proportion of ≥ 90% by weight, based on the total weight of the further low boilers relative to 1,3-butadiene.

6. The process according to any of claims 3 to 5, wherein the polymerization or copolymerization of the 1,3-butadiene is a solution polymerization, especially in the presence of toluene or hexane as a solvent.

7. The process according to any of claims 1 to 6, wherein the stripping column (K1) is configured such that the high boilers are removed down to a residual content of not more than 1000 ppm from the first purified recycle stream (4), which is drawn off via the top thereof.

8. The process according to any of claims 1 to 7, wherein the outgasser column (K3) is configured such that the second purified recycle stream (9) which is drawn off via the top thereof has a 1,3-butadiene content of greater than or equal to 96% by weight, preferably of greater than or equal to 99.5% by weight, based on the total weight of the second purified recycle stream (9).

9. The process according to any of claims 1 to 8, wherein the extractive distillation plant has a stripping column (K4) which is connected upstream of the main scrubber (K2) and is for removal of high boilers from the second substream (5) of the recycle stream (1).

10. The process according to any of claims 1 to 9, wherein a further substream (11) is removed from the recycle stream (1) and is recycled without prepurification into the 1,3-butadiene processing plant (A).

11. The process according to claim 10, wherein the further substream (11) is larger than half of the overall recycle stream (1).

## Revendications

1. Procédé de purification d'un courant de recyclage (1) issu d'une unité de traitement de 1,3-butadiène (A), contenant
- 70 à 99 % en poids de 1,3-butadiène, par rapport au poids total du courant de recyclage (1),
- 0,1 à 10 % en poids de composants de point d'ébullition faible en comparaison du 1,3-butadiène, par rapport au poids total du courant de recyclage (1), et
- 0,1 à 20 % en poids de composants de point d'ébullition élevé en comparaison du 1,3-butadiène, par rapport au poids total du courant de recyclage (1),
la somme du 1,3-butadiène, des composants de point d'ébullition faible et des composants de point d'ébullition élevé dans le courant de recyclage étant de 100 % en poids, et
un courant d'alimentation de 1,3-butadiène pur (2), qui a une teneur en 1,3-butadiène supérieure à 98 % en poids de 1,3-butadiène, par rapport au poids total du courant d'alimentation de 1,3-butadiène pur (2), étant utilisé en tant que point de départ, qui est soumis auparavant à une pré-purification pour la séparation des composants de point d'ébullition élevé en comparaison du 1,3-butadiène dans une colonne de rectification (K1), puis introduit dans l'unité de traitement de 1,3-butadiène (A), **caractérisé en ce que** le courant de recyclage (1) est divisé en deux courants partiels, à savoir
- un premier courant partiel (3), qui est purifié dans la colonne de rectification (K1) pour la pré-purification du courant d'alimentation de 1,3-butadiène pur (2), pour obtenir un premier courant de recyclage purifié (4) qui comprend le courant d'alimentation de 1,3-butadiène pur pré-purifié (2), ainsi que
- un second courant partiel (5), qui est purifié dans une unité de distillation extractive par distillation extractive avec un solvant sélectif (6), l'unité de distillation extractive comprenant
- un épurateur principal (K2), qui est configuré sous la forme d'une colonne d'enrichissement et dans la zone inférieure duquel le second courant partiel (5) du courant de recyclage et dans la zone supérieure duquel le solvant sélectif (6) sont introduits sous forme liquide, le solvant sélectif (6) s'écoulant à contre-courant du second courant partiel (5) du courant de recyclage dans l'épurateur principal (K2) et se chargeant avec du 1,3-butadiène, et duquel un courant de tête (7), contenant des butanes et des butènes, est déchargé, ainsi que
- une colonne de dégazage (K3), dans laquelle le courant de fond (8) de l'épurateur principal (K2) est introduit, et de laquelle un second courant de recyclage purifié (9) est soutiré par la tête, ainsi qu'un courant de fond (10), contenant le solvant sélectif purifié, et
le premier courant de recyclage purifié (4) et le second courant de recyclage purifié (9) étant recyclés dans l'unité de traitement de 1,3-butadiène (A),
à condition que la division du courant de recyclage (1) ait lieu de sorte qu'un débit massique aussi élevé que possible soit attribué au premier courant partiel (3), en maintenant les spécifications pour la somme de tous les courants introduits dans l'unité de traitement de 1,3-butadiène (A), d'une proportion de 1,3-butadiène supérieure ou égale à 90 % en poids, par rapport au poids total de tous les hydrocarbures en C₄ de la somme de tous les courants introduits dans l'unité de traitement de 1,3-butadiène (A).

2. Procédé selon la revendication 1, **caractérisé en ce que** la division du courant de recyclage (1) a lieu de sorte que le premier courant partiel (3) soit plus grand que le second courant partiel (5).

3. Procédé selon la revendication 1 ou 2, **caractérisé en ce que** l'unité de traitement de 1,3-butadiène (A) est une unité pour la polymérisation de 1,3-butadiène ou pour la copolymérisation de 1,3-butadiène avec un ou plusieurs comonomères.

4. Procédé selon la revendication 3, **caractérisé en ce que** le styrène et/ou l'acrylonitrile sont utilisés en tant que comonomère(s).

5. Procédé selon l'une quelconque des revendications 1 à 4, **caractérisé en ce que** le courant de recyclage (1) contient en tant que composants de point d'ébullition faible en comparaison du 1,3-butadiène du méthane, notamment en une proportion ≥ 90 % en poids, par rapport en poids total des autres composants de point d'ébullition faible en comparaison du 1,3-butadiène.

6. Procédé selon l'une quelconque des revendications 3 à 5, **caractérisé en ce que** la polymérisation ou la copolymérisation du 1,3-butadiène est une polymérisation en solution, notamment en présence de toluène ou d'hexane en tant que solvant.

7. Procédé selon l'une quelconque des revendications 1 à 6, **caractérisé en ce que** la colonne de rectification (K1) est configurée de sorte que les composants de point d'ébullition élevé soient séparés jusqu'à une teneur résiduelle d'au plus 1 000 ppm du premier courant de recyclage purifié (4), qui est soutiré par la tête de celle-ci.

8. Procédé selon l'une quelconque des revendications 1 à 7, **caractérisé en ce que** la colonne de dégazage (K3) est configurée de sorte que le second courant de recyclage purifié (9), qui est soutiré par la tête de celle-ci, présente une teneur en 1,3-butadiène supérieure ou égale à 96 % en poids, de préférence supérieure ou égale à 99,5 % en poids, par rapport au poids total du second courant de recyclage purifié (9).

9. Procédé selon l'une quelconque des revendications 1 à 8, **caractérisé en ce que** l'unité de distillation extractive comprend une colonne de rectification (K4) en amont de l'épurateur principal (K2), pour la séparation des composants de point d'ébullition élevé du second courant partiel (5) du courant de recyclage (1).

10. Procédé selon l'une quelconque des revendications 1 à 9, **caractérisé en ce qu'**un courant partiel supplémentaire (11) est séparé du courant de recyclage (1), et est recyclé sans pré-purification dans l'unité de traitement de 1,3-butadiène (A).

11. Procédé selon la revendication 10, **caractérisé en ce que** le courant partiel supplémentaire (11) est supérieur à la moitié du courant de recyclage total (1).
